# EUROPÄISCHE PATENTANMELDUNG

(11) **EP 3 088 017 A1**
(43) Veröffentlichungstag der Anmeldung: **02.11.2016**
(21) Anmeldenummer: 15166042.0
(22) Anmeldetag: 30.04.2015
(51) Int. Cl.: A61M 1/10, A61M 1/12

(54) **ROTOR FÜR EINE FLUIDPUMPE SOWIE VERFAHREN UND GIESSFORM FÜR SEINE HERSTELLUNG**

(71) Anmelder: ECP Entwicklungsgesellschaft mbH, 12247 Berlin (DE)
(72) Erfinder: Sieß, Thorsten, 52074 Aachen (DE); Scheckel, Mario, 14195 Berlin (DE)
(74) Vertreter: Pfenning, Meinig & Partner mbB

(57) **Zusammenfassung**

Gezeigt wird ein Rotor für eine komprimierbare Fluidpumpe, insbesondere eine durch ein Blutgefäß in einen Patientenkörper einführbare Blutpumpe, der ein oder mehrere Förderelemente (15) aufweist und radial zwischen einem ersten, komprimierten und einem zweiten, radial expandierten Zustand komprimierbar und expandierbar ist und der wenigstens teilweise aus einem durch Fasern (10, 11, 13, 18, 19, 55, 56, 62, 63) verstärkten Kunststoff besteht und zur Rotation um eine Rotationsachse (14) vorgesehen ist, wobei der Rotor im ersten, komprimierten Zustand gespannt und im zweiten, expandierten Zustand frei von äußeren Spannungen ist und wobei ein dritter Zustand existiert, den der Rotor (42) im Betriebszustand unter Last einnimmt. Die Fasern verlaufen in dem Rotor im dritten Zustand wenigstens abschnittsweise gestreckt.

## Beschreibung

Die vorliegende Schutzrechtsanmeldung liegt auf dem Gebiet der Mechanik und befasst sich konkret mit Rotoren für Fluidpumpen. Sie ist mit besonderem Vorteil im Bereich der Medizintechnik bei Katheterpumpen einsetzbar.

Im Bereich der Fluidpumpen sind Rotorpumpen bereits in verschiedenen Ausführungsformen als Axialpumpen oder Radialpumpen bekannt. In beiden Fällen wird das zu fördernde Fluid durch eine Rotation eines Rotors und an diesem befestigter Förderelemente beschleunigt, entweder in Axialrichtung oder in Radialrichtung.

Derartige Pumpen können grundsätzlich auch nach dem Stand der Technik bereits komprimiert werden, um sie platzsparend anzuordnen oder zu transportieren. Dies trifft insbesondere auf Katheterpumpen für den medizinischen Anwendungsbereich zu, die oft radial komprimierbar und expandierbar sind, um sie durch einen Katheter bzw. durch Hohlräume im Körper eines Patienten an einen Anwendungsort befördern und dort expandieren zu können, bevor die Inbetriebnahme erfolgt. Solche Pumpen werden beispielsweise zur Unterstützung eines Herzens eines Patienten bei der Blutförderung eingesetzt und zu diesem Zweck durch ein Blutgefäß bis an oder in eine Herzkammer vorgeschoben.

Dabei ergeben sich besondere Herausforderungen durch die geringe Größe des Rotors und zusätzlich seine Komprimierbarkeit. Im expandierten Zustand muss der Rotor trotz seiner Komprimierbarkeit reproduzierbar eine Betriebsform einnehmen, die sich auch im Betrieb bei möglichst hohen Förderdrehzahlen so wenig wie möglich ändert, um eine Verringerung der Effizienz sowie eine Schädigung der zu fördernden Blutbestandteile zu verhindern.

Aus diesem Grund ist bereits der Einsatz verschiedenster Materialien und Materialkombinationen zu dem genannten Zweck erwogen und untersucht worden. Beispielsweise ist aus der WO 2010/063494 A1 der Einsatz verschiedenster Elastomere, auch im Zusammenhang mit einer Faserverstärkung, bereits bekannt.

Aus der WO 2012/007141 A1 ist eine Verstärkung eines Pumpenrotors durch Fasern bekannt, die in orientierter Form, beispielsweise in Radialrichtung, im Rotor angeordnet sein können.

Schließlich ist aus der WO 2012/007140 A1 ein Pumpenrotor mit Verstärkungselementen bekannt, die im Wesentlichen außerhalb der Förderelemente, beispielsweise auf deren Oberflächen, vorgesehen sein können.

Vor dem Hintergrund des Standes der Technik liegt der vorliegenden Erfindung somit die Aufgabe zugrunde, einen Kunststoffrotor der oben genannten Art zu schaffen, der eine minimale Relaxation nach Verformungen zwischen dem komprimierten und dem expandierten Zustand sowie eine möglichst genaue Reproduzierbarkeit seiner Geometrie zumindest im Betriebszustand aufweist.

Die Aufgabe wird durch einen Rotor, ein Verfahren zur Herstellung eines Rotors sowie eine entsprechende Gießform für einen Rotor gelöst.

Es ergibt sich unter anderem dadurch ein Rotor für eine komprimierbare Fluidpumpe, insbesondere eine durch ein Blutgefäß in einen Patientenkörper einführbare Blutpumpe, der ein oder mehrere Förderelemente aufweist und radial zwischen einem ersten, komprimierten und einem zweiten, radial expandierten Zustand komprimierbar und expandierbar ist und der wenigstens teilweise aus einem durch Fasern verstärkten Kunststoff besteht und zur Rotation um eine Rotationsachse vorgesehen ist, wobei der Rotor im ersten, komprimierten Zustand gespannt und im zweiten, expandierten Zustand frei von äußeren Spannungen ist und wobei ein dritter Zustand existiert, den der Rotor im Betriebszustand unter Last einnimmt, dadurch gekennzeichnet, dass die Fasern in dem Rotor im dritten Zustand wenigstens abschnittsweise gestreckt verlaufen.

Derartige Fluidpumpen werden üblicherweise im komprimierten Zustand durch eine Schleuse in ein Blutgefäß, beispielsweise eine Arterie, eingeführt und bis in die Nähe des Herzens oder teilweise bis in ein Patientenherz hinein eingeschoben, um dort expandiert zu werden. Die Erfindung beschränkt sich jedoch nicht auf derartige Blutpumpen, sondern umfasst auch andere Arten von Blutpumpen mit komprimierbaren und expandierbaren Rotoren oder andere Fluidpumpen, die für medizinische oder nichtmedizinische Zwecke in Systeme von Hohlräumen einführbar sind. Im komprimierten Zustand steht der Rotor einer solchen Pumpe üblicherweise unter einer radialen Kompressionsspannung, indem er beispielsweise durch eine äußere Hülle zusammengehalten wird. Die äußere Hülle kann beispielsweise ein Katheter sein, aus dem die Pumpe zur Expansion hinausgeschoben wird. Der Rotor der Pumpe kann jedoch auch durch jede andere Art äußerer Hülle im komprimierten Zustand zusammengehalten werden.

Wird die komprimierende Kraft eliminiert, so entspannt sich die Pumpe, oder zumindest der Rotor der Pumpe, und geht in einen zweiten, expandierten entspannten Zustand über, in dem die radiale Ausdehnung des Rotors sich gegenüber dem ersten, komprimierten Zustand erheblich erweitert.

Wird dann die Pumpe in Betrieb genommen, indem der Rotor rotatorisch angetrieben und ein Fluid gefördert wird, so geht der Rotor in einen Betriebszustand über, der einem Lastzustand unter der Last der geförderten Flüssigkeit entspricht. Besonders dieser Betriebszustand soll bei einer definierten geometrischen Gestalt des Rotors stattfinden, die von der Höhe der Last möglichst unabhängig sein soll. Der Rotor soll zudem beim Übergang zwischen dem zweiten und dem dritten Zustand eine möglichst geringe Formänderung durchlaufen. Jedoch kann der Rotor im dritten Zustand unter der Last der geförderten Flüssigkeit derart verformt werden, dass der Durchmesser des Rotors sich gegenüber dem zweiten, expandierten Zustand vergrößert.

Die Fasern, die das Kunststoffmaterial des Rotors verstärken, sollen derart in der Matrix des Kunststoffmaterials angeordnet sein, dass sie im dritten Zustand wenigstens abschnittsweise gestreckt verlaufen und dadurch den Rotor in seiner Form gegenüber der Last stabilisieren. Die Fasern sollen dabei in Längsrichtung zugbeansprucht werden. Da die Fasern in Längsrichtung praktisch nicht dehnbar sind, führen entsprechende Belastungen in Längsrichtung der Fasern kaum zu einer merklichen Formveränderung der Matrix. Der Rotor oder einzelne Elemente des Rotors, beispielsweise die Förderelemente, kann/können sich somit in der Form vom komprimierten Zustand bis zum Betriebszustand problemlos so lange verändern, bis die Fasern wenigstens abschnittsweise gestreckt sind. An diesem Punkt wird durch die Verankerung der Fasern im Kunststoffmaterial des Rotors zumindest in dem Bereich, in dem die Fasern gestreckt sind, eine Bewegung in Längsrichtung der Fasern auch unter Last praktisch unmöglich gemacht.

Eine besondere Ausgestaltung der Lösung sieht beispielsweise dabei vor, dass die Fasern im dritten Zustand (Betriebszustand) des Rotors wenigstens abschnittsweise in Bereichen des Rotors, in denen dieser eine Dehnspannung aufweist, gestreckt und im Wesentlichen in Richtung der Dehnspannung verlaufen.

Die Fasern sollten wenigstens abschnittsweise in der Verlaufsrichtung angeordnet werden, in der Dehnspannungen im Rotor auftreten, so dass diese Dehnspannungen durch Aufnahme der Längskräfte in den Fasern abgefangen werden und der Rotor sich in seiner Form praktisch nicht bzw. nicht weiter verändert.

Dies erfordert beispielsweise, dass wenigstens ein Teil der Fasern außerhalb der sogenannten neutralen Faser in den Förderelementen des Rotors verläuft. Im Bereich der sogenannten neutralen Faser und/oder neutralen Fläche in der Nomenklatur der Biegemechanik würde eine zugfeste Faser keine zusätzliche Stabilisierung des Rotors erbringen. Die biegetechnisch neutrale Faser ist bei den hier beschriebenen Rotoren in der Regel eine Fläche, weshalb im Folgenden zur besseren Klarheit der Begriff biegeneutrale Fläche bzw. neutrale Fläche verwendet wird.

Es kann deshalb vorgesehen sein, dass mehr als die Hälfte der Fasern in Bereichen des Rotors und/oder der Förderelemente angeordnet sind, in denen dieser/diese im Betriebszustand Dehnspannungen aufweist/aufweisen. Besonders vorteilhaft ist es dabei, wenn viele Fasern in dem Bereich des Rotors oder eines Förderelements angeordnet sind, der unter Last Dehnspannungen aufweist.

Zudem kann vorgesehen sein, dass bei dem Rotor zwischen dem zweiten, expandierten Zustand in dem dritten Betriebszustand bezüglich der äußeren Form im Wesentlichen keine Unterschiede bestehen.

Zu einer besonders guten Stabilisierung des Rotors insgesamt kann beispielsweise auch vorgesehen sein, dass Fasern vorgesehen sind, die sich in radialer Richtung über die Rotationsachse hinaus erstrecken. Damit werden einzelne Förderelemente nicht nur gegen eine Biegung in sich stabilisiert, sondern auch gegenüber einer Schwenkbewegung um einen rotationsachsennahen Teil des Rotors, beispielsweise eine Nabe.

Zudem kann vorteilhaft vorgesehen sein, dass im Betriebszustand wenigstens ein Teil der Fasern, insbesondere die Mehrheit der Fasern, abschnittsweise gerade verlaufen. Zwischen den Abschnitten, in denen die Fasern gerade verlaufen, können andere gerade Abschnitte oder auch gekrümmte Abschnitte der Fasern vorgesehen sein.

Es kann auch vorgesehen sein, dass im Betriebszustand wenigstens ein Teil der Fasern, insbesondere die Mehrheit der Fasern, entlang der Längsrichtung des jeweiligen Förderelementes mit einer geringeren Krümmung verlaufen als die neutrale Fläche des jeweiligen Förderelementes im Sinne der Festigkeitslehre. Durch diese Anordnung der Fasern wird sichergestellt, dass die Verstärkungsfasern einerseits im Betriebszustand bereits stark gestreckt sind und dass sie andererseits außerhalb der neutralen Fläche der Förderelemente verlaufen und somit in solchen Bereichen, die unter Belastungen Dehnspannungen aufweisen können.

Es kann zudem auch vorgesehen sein, dass der Kunststoff des Rotors eine Shore-Härte <100 D, insbesondere < 80 D, aufweist. Durch die geringe Shore-Härte, die beispielsweise auch kleiner als 80 D gewählt werden kann, ergibt sich bei starker Biegung oder Knickung des Materials für die eingebetteten Fasern die Möglichkeit, in das nachgiebige Material der Basis auszuweichen und dadurch den Krümmungsradius nach unten zu begrenzen. Damit ergibt sich eine Verringerung der Bruchgefahr für die Fasern.

Zudem kann bei einem solchen Rotor auch vorgesehen sein, dass ein erster Anteil von mehr als 30%, insbesondere mehr als 50%, der Fasern im expandierten Zustand des Rotors im Wesentlichen gestreckt von einem der Rotorachse am nächsten liegenden Abschnitt zu einem der Rotationsachse ferner liegenden zweiten Abschnitt verläuft. Durch diese Positionierung, Ausrichtung und Formung der Fasern wird eine Überstreckung des Rotors über den expandierten Zustand hinaus wirkungsvoll vermieden.

Eine Rotorkonstruktion kann auch dadurch ausgestaltet werden, dass ein erster Anteil der Fasern, der mehr als 30% der Fasern, insbesondere mehr als 50%, der Fasern beträgt, eine Länge aufweist, die mindestens 30%, insbesondere mindestens 50%, der Maximalhöhe der Förderelemente, gemessen in Radialrichtung des Rotors im expandierten Zustand, entspricht. Hierdurch ergeben sich Stabilisierungskräfte, die größere Teile des Rotors umfassen und diesen insgesamt stabilisieren.

Dazu kann außerdem vorgesehen sein, dass der erste Anteil der Fasern im Betriebszustand im Wesentlichen senkrecht zur Rotationsachse verläuft. Durch diese Ausrichtung der Fasern können Dehnspannungen im Rotor, die durch die Last des zu fördernden Fluids erzeugt werden, optimal aufgenommen werden.

Weiter kann die Bauart des Rotors dadurch ausgestaltet werden, dass der Durchmesser der Fasern, insbesondere des ersten Anteils der Fasern, kleiner als 40 Mikrometer ist. Durch einen derart klein gestalteten Durchmesser der Fasern können geringe Biegeradien erreicht werden, so dass die Bruchgefahr für die Fasern weiter vermindert ist.

Zudem kann vorgesehen sein, dass die Oberfläche der Fasern mit einem Haftvermittler versehen ist. Dadurch wird eine gute Verankerung der Fasern im Kunststoffmaterial der Matrix erreicht, was zu einer weiteren Verbesserung bei der Aufnahme von Dehnspannungen durch die Fasern führt.

Die Erfindung bezieht sich zudem auf ein Verfahren zur Herstellung eines Rotors nach Anspruch 1 oder einem der folgenden mittels eines Gießverfahrens, insbesondere eines Spritzgießverfahrens, bei dem das Material der Förderelemente in Radialrichtung in Bezug auf die Rotorachse in die Volumina der Förderelemente derart eingebracht wird, dass der Gießwerkstoff in das Volumen jedes einzelnen Förderelementes jeweils in Radialrichtung einströmt.

Bei einem solchen Verfahren kann das Gussmaterial beispielsweise in Axialrichtung im zentralen Bereich des Rotors, beispielsweise im Volumen einer Rotornabe, eingespritzt und von dort radial in die einzelnen Förderelemente hinein verteilt werden. Jedes einzelne Förderelement wird dann in einer radial gerichteten Flussrichtung des Materials von innen nach außen gefüllt. Es ist jedoch auch eine Füllung von den radial außen liegenden Enden der Förderelemente radial nach innen, auf die Rotorachse hin gerichtet, möglich.

Die in dem Gussmaterial vorhandenen Fasern, die beispielsweise aus Glas, jedoch auch aus Kohlenstoff bzw. Polycarbonat bestehen können, werden durch den Strom des Matrixmaterials mitgenommen und vorwiegend in Flussrichtung des Materials ausgerichtet.

Eine für die Herstellung des beschriebenen Rotors und für das beschriebene Verfahren optimierte Gießform soll ebenfalls vorgestellt werden. Diese zeichnet sich dadurch aus, dass bei den Volumina der Förderelemente an deren radial verlaufenden Kanten Überströmkanäle vorgesehen sind, um einen störungsfreien Fluss des Gießwerkstoffs in Radialrichtung zu ermöglichen.

Durch die an den Kanten vorgesehenen Überströmkanäle wird die Ausbildung von Verwirbelungen mit lokalen Rückströmungen im Bereich der Kanten der Förderelemente verhindert, so dass eine weitgehend laminare Strömung ausgebildet werden kann. Hierdurch werden die Fasern in überwiegend gestreckter Lage in die Matrix eingebettet. Dies ist insbesondere deshalb sinnvoll, da die Gießform den Guss eines Rotors im zweiten, expandierten Zustand, d. h. frei von äußeren Spannungen, ermöglicht.

Zusätzlich zu den an den seitlichen Kanten der Förderelemente vorgesehenen Überströmkanälen können auch an den radial außen gelegenen Enden der Volumina in der Gießform Überströmkanäle ausgebildet sein, die das Abströmen an den radial außen gelegenen Enden der Förderelemente erlauben. Das überschüssige Gussmaterial kann nach wenigstens teilweisem Erstarren des Rotors in der Gießform entfernt werden.

Es kann bei der Anordnung der Fasern im Volumen der Förderelemente gezielt eine Beabstandung von der biegeneutralen Fläche angestrebt werden, um bei der Belastung durch den Fluidgegendruck im Betriebszustand eine besondere Stabilisierung bei Ausbiegung der Förderelemente in eine Richtung durch Streckung der Fasern zu erreichen. Jedenfalls sollten die Fasern vorteilhaft im Inneren der Förderelemente verlaufen und einen bestimmten Mindestabstand zu den äußeren Begrenzungsflächen der Förderelemente einhalten.

Es können in dem Rotor auch mehrere Gruppen von Fasern unterschiedlicher Länge vorgesehen sein, wobei wenigstens eine Gruppe eine bestimmte Mindestlänge aufweist, während die Fasern von einer oder mehreren Gruppen kürzer sind oder eine Längenverteilung aufweisen, die nur vernachlässigbar wenige Fasern oberhalb einer Faserlänge aufweist, die unterhalb einer typischen Faserlänge des ersten Anteils der Fasern liegt. Typisch liegt die Durchschnittslänge der kürzeren Fasern bei weniger als einem Drittel der Länge des ersten Anteils der Fasern.

Der Rotor kann vorteilhaft dadurch ausgestaltet werden, dass jede Faser des ersten Anteils der Fasern in ihrem Verlauf höchstens 45° in Axialrichtung und/oder Azimutalrichtung von einer radial auf die Rotorachse (14) ausgerichteten Lage abweicht. Die Fasern verlaufen dann in einer Fläche, in der auch die gesamte Rotationsachse des Rotors verläuft, so dass die Fasern sich beispielsweise direkt von der Rotationsachse ausgehend zunächst senkrecht radial nach außen erstrecken können. Jedoch ist auch eine Ausrichtung der Fasern von der Rotationsachse radial in einem Winkel zwischen 45° und 90° zur Rotationsachse möglich. In einer Ausgestaltung weist die Erstreckung der Fasern jedenfalls keine oder nur geringe azimutale (in Umfangsrichtung verlaufende) Ausrichtung auf.

Der Gegenstand kann weiterhin dadurch ausgestaltet sein, dass die Förderelemente aus einem Schaumstoff bestehen. Dabei ist insbesondere an einen geschlossenporigen Schaumstoff gedacht, der durch die Verstärkungsfasern effektiv stabilisierbar ist und der dennoch einfach und in ausreichendem Maß komprimierbar ist. Bei der Kompressionsbewegung ist gerade bei einem Schaumstoff ein Ausweichen der Fasern zur Verhinderung der Unterschreitung eines kritischen Biegeradius besonders einfach möglich. Üblicherweise weisen derartige Schaumstoffe im Inneren des Volumens der Förderelemente entsprechende Poren auf, sind jedoch an den äußeren Begrenzungsflächen praktisch vollständig geschlossen.

Im Folgenden wird die Neuerung anhand von Ausführungsbeispielen in Figuren einer Zeichnung gezeigt und nachfolgend erläutert. Dabei zeigt
- Fig. 1: schematisch in einem Schnitt eine Katheterpumpe, die in eine Herzkammer eines Patienten eingeführt ist,
- Fig. 2a: einen Ausschnitt aus einem Rotor einer Katheterpumpe zur Förderung von Blut,
- Fig. 2b: eine Querschnittsdarstellung eines entspannten Rotors mit einer beispielhaft dargestellten Faser,
- Fig. 2c: die Querschnittsdarstellung eines Rotors im dritten Zustand, dem Betriebszustand,
- Fig. 2d: die Querschnittsdarstellung eines Rotors im komprimierten Zustand,
- Fig. 2e: eine Querschnittsdarstellung eines Förderelementes mit Markierung der biegetechnisch neutralen Fläche sowie mit beispielhaft dargestellten Verstärkungsfasern,
- Fig. 3: eine schematische Darstellung einer Ebene, die die Rotationsachse des Rotors enthält,
- Fig. 4: einen Ausschnitt aus einem planen Förderelement mit einer Faser zur Verstärkung,
- Fig. 5a: die Ausrichtung einer Faser beim Gießvorgang,
- Fig. 5b: eine Seitenansicht des Abschnitts aus Figur 4,
- Fig. 6: den Abschnitt eines Förderelements wie in Figur 4 und 5 gezeigt nach einem Knickvorgang,
- Fig. 7: eine schematische Ansicht eines Gießwerkzeugs für einen Rotor, wobei der Gießvorgang durch Strömungspfeile angedeutet ist,
- Fig. 8: eine Gießform für einen Rotor mit einer verglichen mit Figur 7 umgekehrten Einspritzrichtung sowie
- Fig. 9: eine Gießform, bei der Maßnahmen gegen eine Verwirbelung des Spritzgusswerkstoffs im Volumen der Förderelemente getroffen sind.

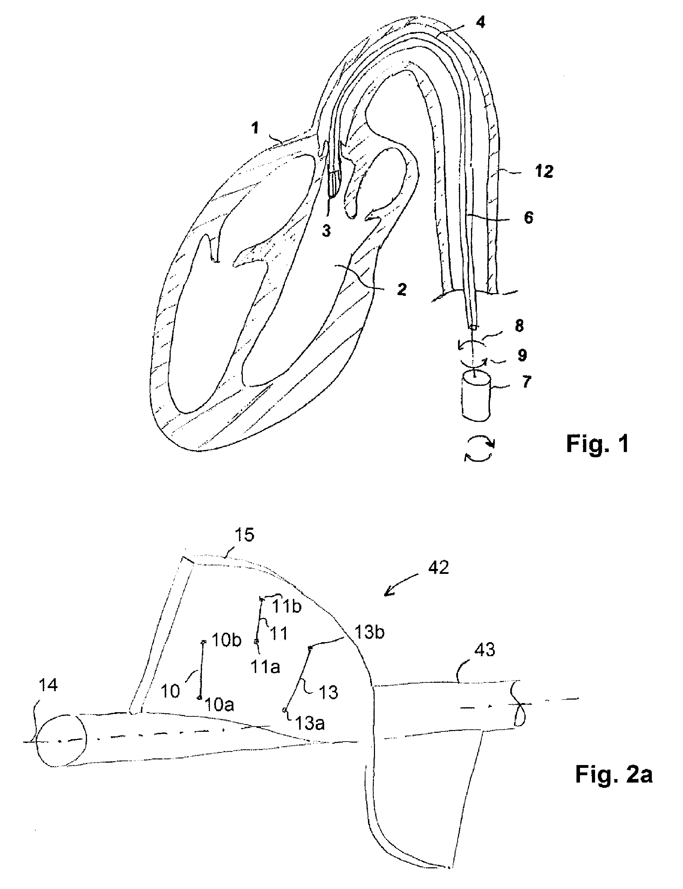

Figur 1 zeigt im Querschnitt das Herz 1 eines Patienten mit mehreren Herzkammern, wobei die Herzkammer 2 mit der Aorta 12 in Verbindung steht. Durch die Aorta 12 ist ein Katheter 4 bis in die Herzkammer 2 vorgeschoben, wobei am Ende des Katheters 4 ein Pumpenkopf 3 mit einer Rotationspumpe angeordnet ist. Die Rotationspumpe ist mittels einer durch den Katheter 4 verlaufenden drehbaren Welle 6 antreibbar, die innerhalb des Pumpenkopfes 3 mit einem Pumpenrotor 42 verbunden ist. Der Pumpenrotor rotiert in einem nicht im Einzelnen dargestellten Gehäuse des Pumpenkopfes 3.

Die biegsame Welle 6 ist mit einem Motor 7 verbunden, der beispielsweise außerhalb des Patientenkörpers angeordnet ist. Das Drehmoment kann beispielsweise über eine Magnetkopplung von dem Motor 7 in beiden Drehrichtungen 8, 9 auf die Welle 6 übertragen werden.

Der Katheter 4 wird üblicherweise über eine Schleuse vom Körperäußeren durch die Haut und Gewebe sowie die Gefäßwand in die Aorta 12 hinein und in dieser vorgeschoben.

Die Pumpe saugt in der Herzkammer 2 Blut an und fördert dies in die Aorta 12. Hierdurch kann die Herzpumpe die Funktion des Herzens 1 entweder unterstützen oder zumindest zeitweise ersetzen.

Neben der in der Figur dargestellten Katheterpumpe mit mechanischem Antrieb sind auch andere Pumpen, insbesondere zur intrakorporalen Anwendung, Gegenstand dieses Schutzrechts, beispielsweise Pumpen mit hydraulischem oder elektrischem Antrieb, und dort auch Pumpen, bei denen der Antrieb innerhalb des menschlichen Körpers ist.

Die Pumpe wird mitsamt dem Pumpenkopf, dem Pumpengehäuse und dem Rotor zur Verschiebung in der Aorta radial komprimiert und beispielsweise innerhalb des Katheters 4 verschoben. Die Pumpe kann dann aus dem Katheter 4 axial herausgeschoben werden und sich radial entfalten, d. h. expandiert werden. Dabei werden hohe Anforderungen an die Materialien des Pumpengehäuses und insbesondere des Pumpenrotors gestellt: Die Förderelemente des Pumpenrotors weisen eine sehr geringe Wandstärke auf, müssen aber dennoch auch bei hohen Rotationdrehzahlen formstabil bleiben und in reproduzierbarer Weise Blut fördern.

Zu diesem Zweck sind in die Matrix des Kunststoffs, aus dem der Rotor besteht, Verstärkungsfasern (Fasern) eingebettet, die beispielsweise als Glasfasern oder Polycarbonatfasern ausgestaltet sein können. Solche Fasern sind in Figur 2a in drei Einzelbeispielen dargestellt. Es sind die drei Fasern 10, 11, 13 gezeigt, von denen jede ein erstes Ende oder einen ersten Abschnitt 10a, 11a, 13a aufweist, das/der der Rotationsachse 14 näher ist als das jeweilige zweite Ende / der jeweilige zweite Abschnitt 10b, 11b, 13b der Fasern 20, 11, 13.

Die Fasern 10, 11, 13 erstrecken sich im Wesentlichen von der Rotationsachse oder einem Punkt in der Nähe der Rotationsachse 14 des Rotors radial nach außen weg. Dabei ist es nicht notwendig, dass der Rotor 42, wie im dargestellten Beispiel, eine Nabe 43 aufweist. Das wendelartige Förderelement 15 kann auch eine derartige Eigenstabilität aufweisen, dass eine Rotornabe nicht notwendig ist.

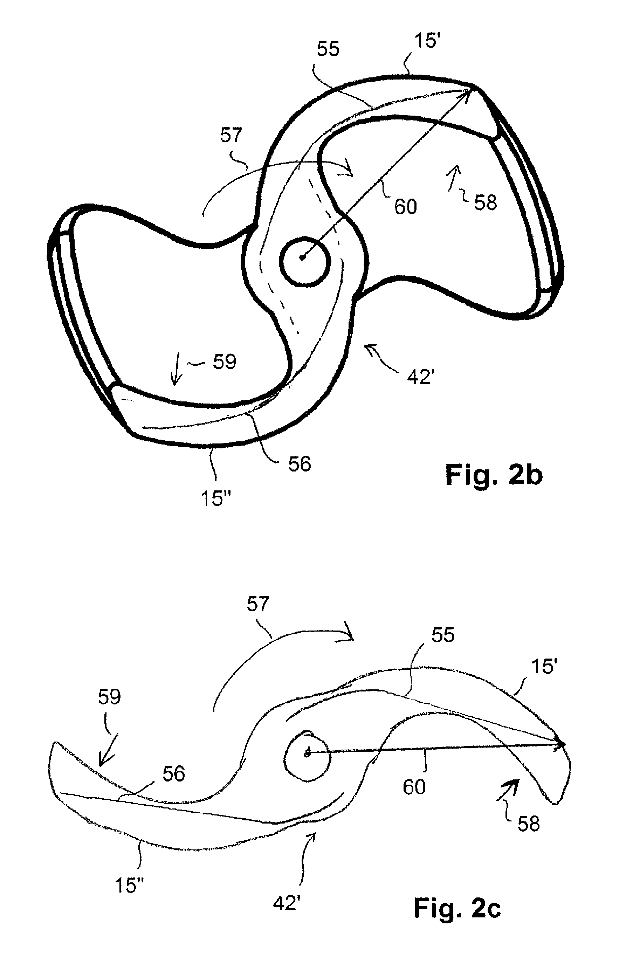

Grundsätzlich kann die Kunststoffmatrix des Förderelements oder der Förderelemente 15 mit Fasern verstärkt sein, die in Bezug auf Länge und/oder Dicke und/oder Orientierung ungleichmäßig verteilt und angeordnet sind. Ein Aspekt ist, dass ein gewisser Mindestanteil der Fasern im expandierten Zustand des Rotors, der in Figur 2b dargestellt ist, im Wesentlichen gestreckt von der Rotationsachse weg verläuft. Im Betriebszustand, der dem dritten Zustand entspricht und der in Figur 2c dargestellt ist, sind diese Fasern gestreckt und stehen zudem unter Zugspannung, die durch die Last der geförderten Flüssigkeit erzeugt wird. Diese Zugspannung führt allerdings wegen der Längsfestigkeit der Fasern nicht zu einer wahrnehmbaren Längung und somit auch praktisch nicht zu einer Formänderung des Rotors gegenüber dem spannungslosen expandierten Zustand bzw. zu keiner weiteren Verformung oberhalb eines bestimmten Betriebspunktes. Der Anteil der Fasern, der die genannte Bedingung erfüllt, von der Gesamtmenge der Fasern, die in den Kunststoff des Rotors eingebettet sind, sollte wenigstens 30% oder vorteilhaft 50% oder noch vorteilhafter beispielsweise 70%, gemessen in Volumen- oder Massenprozent der Fasern oder auch in der Zahl der Fasern, darstellen. Dabei ist vorteilhaft eine gewisse Mindestlänge der Fasern gegeben, beispielsweise etwa wenigstens 20% oder wenigstens 40% oder 50% des Radius des Rotors. Die Fasern können während des Füllvorgangs der Form leicht aus der axialen Ausrichtung, die der Einfüllrichtung axial in die Nabe 43 entspricht, in die radiale Position der Förderelemente 15 wechseln ohne hierfür gebogen zu werden, da der Winkel zwischen dem Förderelement 15 und der Nabe 43 mit < 30° oder bevorzugt < 20° relativ flach verläuft. Somit werden die Fasern bei der radialen Befüllung der Förderelemente 15 einfach von der sie umgebenden Matrix mitgenommen und mit dem Materialfluss im Förderelement radial gemäß Figur 2a ausgerichtet.

Weitere vorteilhafte Eigenschaften der Fasern sind eine gewisse Maximaldicke, wobei ein Durchmesser von höchstens 40 µm vorteilhaft sein kann, um bei einer starken Biegung der Fasern keinen Bruch der Fasern selbst zu erzeugen. Mit ca. 40 µm hingegen sind die Fasern biegesteif genug, um die sie umgebende Matrix nach erfolgter Deformation wieder in den Ausganszustand zurück zu holen und ein nachhaltiges Kriechen der Matrix unter dauerhafter Biegebelastung zu verhindern. Ferner sind Fasern mit 40 µm Durchmesser druck- und zugsteif, so dass sie bei einer Anordnung außerhalb des biegeneutralen Bereiches ebenfalls ein rückstellendes Moment erzeugen und einer bleibenden Deformation entgegen wirken.

Die Fasern können für eine Verbesserung der Verbindung mit der Matrix mit einem Haftvermittler beschichtet sein.

Figur 2b zeigt im Querschnitt einen Rotor 42' mit Förderelementen 15', 15" im entspannten, expandierten Zustand. Die Fasern 55, 56 weisen in diesem Zustand eine möglichst gestreckte Form auf, so dass sie einer weitergehenden Verformung des Rotors durch ihre Längssteifigkeit einen Widerstand entgegensetzen.

Der Pfeil 57 zeigt die Rotationsrichtung des Rotors im Betriebszustand. Die Pfeile 58, 59 zeigen die auf die Förderelemente 15', 15" wirkende Last, d. h. den durch die Förderbewegung wirkenden Fluidgegendruck auf die Förderelemente. In Figur 2b ist die Sehne 60 eingezeichnet, die die Höhe des Förderelements 15' in radialer Richtung von der Rotationsachse aus gemessen bezeichnet.

Die Sehne 60 ist auch in Figur 2c eingezeichnet, welche den Rotor 42' im dritten Zustand, dem Betriebszustand, zeigt. Die Verstärkungsfasern 55, 56 stehen durch die Last 58, 59 unter Zugspannung und nehmen die Dehnungsspannungen in den Förderelementen 15', 15" auf. Die Länge der Sehne 60, d. h. die Höhe der Förderelemente, ist im Betriebszustand gegenüber dem zweiten Zustand gleichbleibend oder um ein geringes Maß erhöht, jedoch nicht verringert.

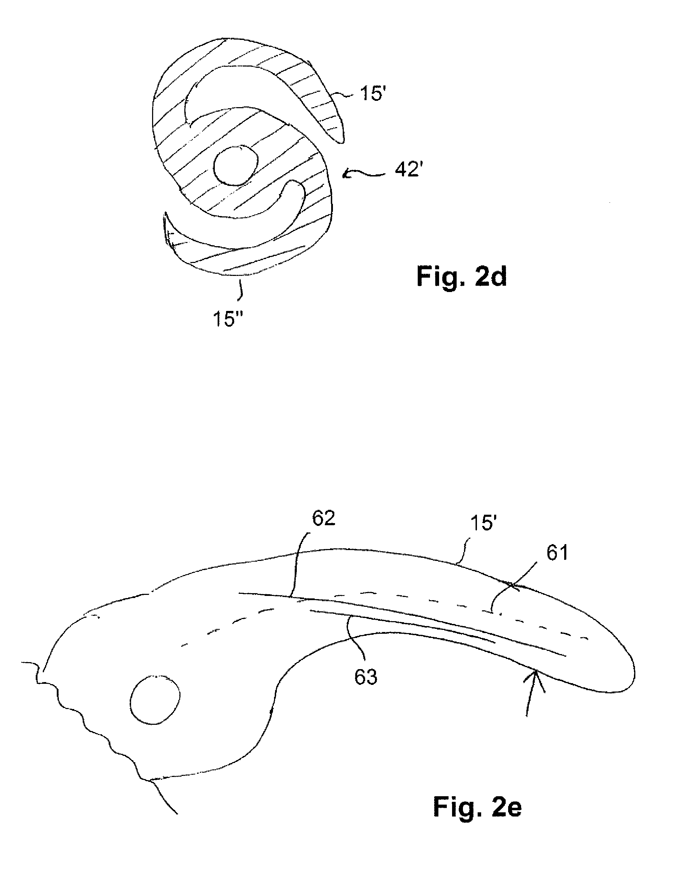

Figur 2d zeigt den Rotor 42' in komprimierter Form, in der die Förderelemente an die Rotornabe angeklappt sind. In dieser Stellung kann die Pumpe durch einen engen Kanal an den Betriebsort gebracht werden.

In Figur 2e ist ein Betriebszustand des Förderelements 15' im Querschnitt detaillierter dargestellt. Es ist die "biegeneutrale Faser" oder "biegeneutrale Fläche" durch die gestrichelte Linie 61 dargestellt, d. h. die Linie oder Fläche, die beim Biegen des Förderelements 15' unter Last weder eine Längung noch eine Stauchung erfährt. Eine Mehrzahl von Verstärkungsfasern, beispielhaft mit 62, 63 bezeichnet, liegt auf der Seite der Linie/Fläche 61, auf der im Betriebszustand eine Dehnspannung entsteht. Die Fasern sind dort abschnittsweise gestreckt und verlaufen gerade sowie im Wesentlichen parallel zu der Linie/Fläche 61. Hierdurch können sie Dehnspannungen effektiv aufnehmen.

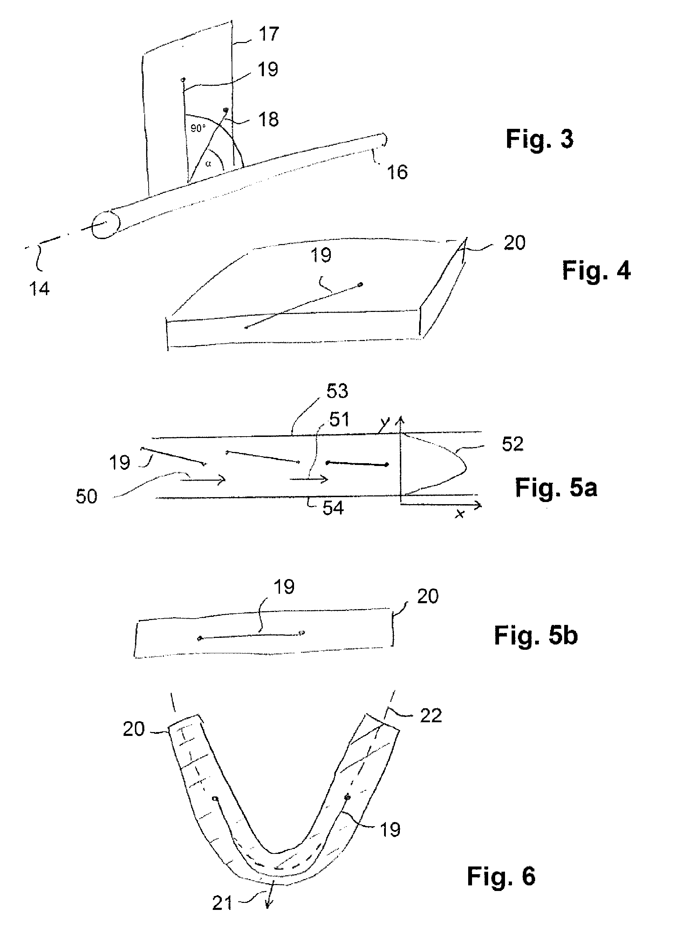

Anhand der Figur 3 soll weiter die mögliche Orientierung der Fasern erläutert werden. Die Rotornabe ist in Figur 3 mit 16 bezeichnet, und die Rotationsachse mit 14. Es ist eine Ebene 17 anhand eines ausgeschnittenen Rechtecks dargestellt, wobei die Ebene 17 die Rotationsachse 14 enthält, d. h., die Rotationsachse 14 verläuft vollständig in der Ebene 17. Es sei bemerkt, dass diese Darstellung vereinfacht ist, da üblicherweise die Förderelemente wendelförmig sind, so dass die Fläche 17 dann nicht mehr eben, sondern gekrümmt ist.

Es sind beispielhaft zwei Fasern 18, 19 eingezeichnet, die beide in der Ebene 17 im Wesentlichen radial in Bezug auf die Rotationsachse 14 verlaufen. Die Faser 18 verläuft in einem Winkel α zur Rotationsachse 14, teilweise in Axialrichtung, wobei der Winkel α vorteilhaft zwischen 45° und 90° liegt. Die Faser 19 ist derart ausgerichtet, dass sie senkrecht auf der Rotationsachse 14 steht. Ein reales Schaufelblatt ist in drei Dimensionen wendelförmig gebogen, so dass in vielen Fällen eine begrenzte Erstreckung der Fasern in Azimutalrichtung hinzukommt.

Die einzelnen Fasern müssen nicht derart positioniert sein, dass sie im Bereich der Rotationsachse 14 oder der Rotornabe 16 ihren ersten Anfangspunkt/ Endpunkt haben. Sie können auch so angeordnet sein, dass sie zwischen zwei Endpunkten verlaufen, die beide von der Rotorachse 14 und/oder von der Rotornabe 16 radial beabstandet sind. Sie können sich aber auch von einem ersten radial, außen liegenden Schaufelrand bis maximal zu einem zweiten, radial gegenüber liegenden Schaufelrand, jedenfalls über die Achse hinweg, erstrecken.

In Figur 4 ist schematisch ein Ausschnitt 20 aus einem Förderelement dargestellt, wobei der Ausschnitt 20 quaderförmig gestaltet ist. In dem Abschnitt 20 ist eine Faser 19 dargestellt.

In Figur 5a wird deutlich gemacht, wie die zentrale Ausrichtung der Faser 19 erreicht wird. Das bevorzugt laminare Strömungsprofil beim Befüllen der Gießform zwischen den Begrenzungswänden 53 und 54 hat die höchste Strömungsgeschwindigkeit in der Mitte und die niedrigste Geschwindigkeit in der Nähe der Bewandung. Die nun zunächst windschief liegende Faser 19 ist in drei von links nach rechts nacheinander erreichten Winkelpositionen dargestellt und wird durch die Materialströmung, angedeutet durch die Pfeile 50, 51, infolge der Geschwindigkeitsverteilung in der Gießform in Richtung der Mitte des Strömungsprofils gezogen. Die Geschwindigkeitsverteilung der Strömung ist in dem Graphen 52 des Diagramms mit den Achsen x (=̂ Geschwindigkeit) und y (=̂ Ortskoordinate in der Gießform) dargestellt. Figur 5b zeigt eine Seitenansicht, wobei das Material des Abschnitts 20 transparent dargestellt ist, so dass der Verlauf der Faser 19 etwa in der Mitte zwischen den Begrenzungsflächen des Förderelements sichtbar ist.

Anhand der Figur 6 soll das Verhalten der Faser bei einer starken Biegung oder Knickung des Förderelements beschrieben werden.

Figur 6 zeigt den Abschnitt 20 des Förderelements nach einer Knickung. Die Faser 19 ist ebenfalls verformt. Durch eine gewisse Eigensteifigkeit der Faser kann diese jedoch wegen der Nachgiebigkeit des Materials des Förderelements beim Knicken nach außen in Richtung des Pfeils 21 ausweichen, um einen möglichst großen Krümmungsradius der Faser zu erreichen und damit einem Bruch der Faser entgegenzuwirken. Im Bereich der Knickstelle ist damit die Faser 19 aus der Mitte zwischen den Begrenzungswänden des Förderelements für die Zeitdauer der Knickung ausgelenkt. In Figur 6 ist die Mittelebene des Förderelements der Übersichtlichkeit halber wenigstens abschnittsweise durch eine gestrichelte Linie 22 dargestellt. Um diesen Effekt zu erreichen, ist eine Weichheit der Kunststoffmatrix des Rotors vorteilhaft, die einer Shorehärte < 100 D, insbesondere < 80 D, entspricht.

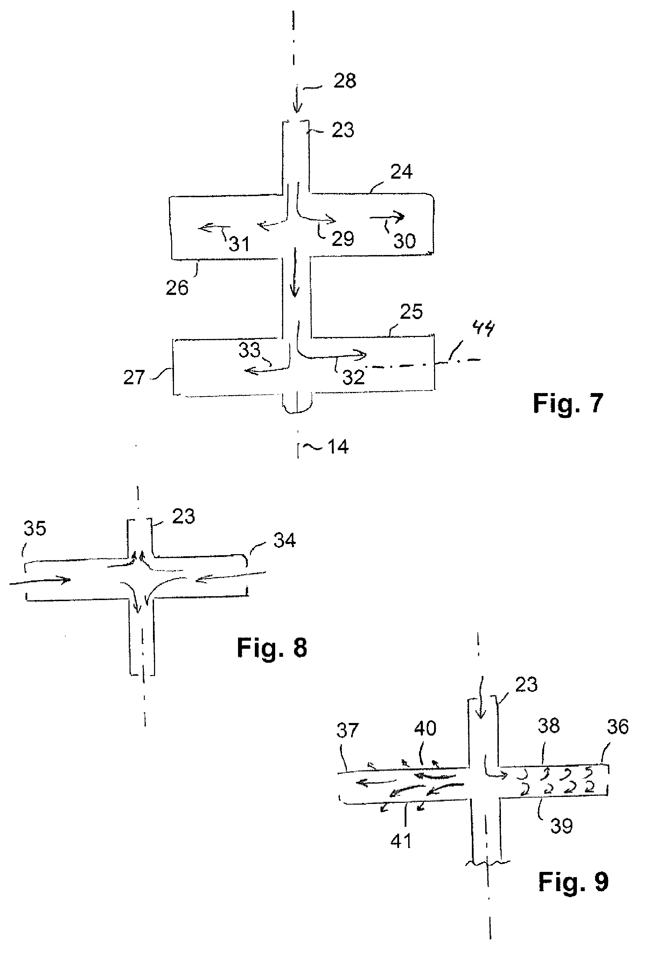

Anhand der Figur 7 soll auf die erfindungsgemäße Gestaltung eines Gusswerkzeugs / einer Gießform für einen erfindungsgemäßen Rotor eingegangen werden.

Es ist in Figur 7 ein Längsschnitt der Spritzgießform gezeigt, wobei der Bereich, der das Volumen der Rotornabe umfasst, mit 23 bezeichnet ist und die Volumina der einzelnen Förderelemente mit 24, 25, 26, 27 bezeichnet sind. In Figur 7 ist zudem eine Einspritzrichtung durch den Pfeil 28 angedeutet. Durch die weiteren Pfeile 29, 30, 31, 32, 33 ist angedeutet, dass der Spritzgusswerkstoff axial entlang der Rotationsachse 14 des entstehenden Rotors einströmt, und von dort aus radial nach außen in die Volumina der Förderelemente hinein. Beispielhaft ist die Längsachse eines der Förderelemente mit einer strichpunktierten Linie angedeutet und mit 44 bezeichnet. Werden in den Spritzgusswerkstoff entsprechend lange Fasern in ausreichender Anzahl eingebracht, so orientieren diese sich nach der Hauptströmungsrichtung des Werkstoffs und verbleiben beim Erstarren des Materials an Ort und Stelle. Verdrängte Luft und überschüssiges Gießmaterial können am radial äußeren Ende der Förderelemente durch Öffnungen 45 abströmen.

In Figur 8 ist eine umgekehrte Einspritzrichtung angedeutet, wobei der Spritzgusswerkstoff von den radial äußeren Enden 34, 35 der Förderelemente radial nach innen zum Volumen des Rotors 23 eingespritzt wird. Auch in diesem Fall können lange Fasern mit eingebracht werden, die sich in der gemäß der Erfindung beabsichtigten Art und Weise orientieren und anordnen.

Anhand der Figur 9 wird gezeigt, dass bei schmalen Förderelementen 36, 37 die radial von innen nach außen strömende Vergussmasse an den Kanten 38, 39 des Volumens des jeweiligen Förderelements durch die Reibung verwirbelt, so dass sich keine laminare Strömung der Vergusswerkstoffs beim Einströmen ergibt.

Auf der linken Seite in der Darstellung der Figur 9 ist eine Gussformvariante dargestellt, die im Bereich der Kanten 40, 41 Überströmöffnungen oder Überströmschlitze aufweist, durch die ein Teil des Spritzgusswerkstoffs in Axialrichtung des Rotors abströmen kann, so dass die auf der rechten Seite der Figur 9 dargestellte Verwirbelung nicht entsteht. Im mittleren Bereich des Förderelements 37 ergibt sich damit eine quasi laminare Strömung, so dass die eingebrachten Fasern sich dort in einem Bereich um die biegetechnisch "neutrale Faser" oder neutrale Fläche herum in gestreckter Form anordnen können, ohne dass sie durch Beeinflussung der Strömung des Spritzgusswerkstoffs verformt werden. Nach dem Erstarren können die Teile des Spritzgusswerkstoffs, die durch die Öffnungen an den Kanten 40, 41 der Volumina der Förderelemente in der Spritzgießform ausgetreten sind, entfernt werden, beispielsweise durch Abschneiden. Gleiches kann, wie in Figur 7, an den Außenkanten der Förderelemente erfolgen. Hier sind zwei Überströmkanäle 45 vorgesehen, durch die der Kunststoff ohne Fasern entweichen kann.

Durch eine asymmetrische Verteilung der Abströmöffnungen, beispielsweise einen größeren Abströmquerschnitt in der Nähe des Bereiches des Förderelements, das im Betrieb dem Fluidgegendruck der Flüssigkeit ausgesetzt ist, ergibt sich dort eine stärkere Strömung des Gießwerkstoffs, so dass dort mehr Fasern als auf der der "neutralen Fläche" gegenüberliegenden Seite eingelagert werden, mit dem Ergebnis, dass die Dehnspannungen durch eine hohe Anzahl von Fasern aufgenommen werden können.

Durch die oben angegebenen Merkmale, insbesondere durch Gestaltung des Rotors und Einbringen geeigneter Fasern, wird eine stabile Gestaltung eines Rotors mit ausreichender Formtreue auch nach teilweiser Überstreckung oder häufiger Wechselbelastung oder konstant anliegender Biegebelastung erreicht. Durch die erläuterten Herstellungsverfahren und die dargestellte Spritzgießform wird eine sinnvolle und vorteilhafte Herstellungsmöglichkeit für den vorgestellten Rotor aufgezeigt.

## Patentansprüche

1. Rotor für eine komprimierbare Fluidpumpe, insbesondere eine durch ein Blutgefäß in einen Patientenkörper einführbare Blutpumpe, der ein oder mehrere Förderelemente (15) aufweist und radial zwischen einem ersten, komprimierten und einem zweiten, radial expandierten Zustand komprimierbar und expandierbar ist und der wenigstens teilweise aus einem durch Fasern (10, 11, 13, 18, 19, 55, 56, 62, 63) verstärkten Kunststoff besteht und zur Rotation um eine Rotationsachse (14) vorgesehen ist, wobei der Rotor im ersten, komprimierten Zustand gespannt und im zweiten, expandierten Zustand frei von äußeren Spannungen ist und wobei ein dritter Zustand existiert, den der Rotor (42) im Betriebszustand unter Last einnimmt, **dadurch gekennzeichnet, dass** die Fasern in dem Rotor im dritten Zustand wenigstens abschnittsweise gestreckt verlaufen.

2. Rotor nach Anspruch 1, **dadurch gekennzeichnet, dass** die Fasern (10, 11, 13, 18, 19, 55, 56, 62, 63) im dritten Zustand (Betriebszustand) des Rotors wenigstens abschnittsweise in Bereichen des Rotors (42), in denen dieser eine Dehnspannung aufweist, gestreckt und im Wesentlichen in Richtung der Dehnspannung verlaufen.

3. Rotor nach Anspruch 1 oder 2, **dadurch gekennzeichnet, dass** mehr als die Hälfte der Fasern (10, 11, 13, 18, 19, 55, 56, 62, 63) in Bereichen des Rotors und/oder der Förderelemente angeordnet sind, in denen dieser/diese im Betriebszustand Dehnspannungen aufweist/aufweisen.

4. Rotor nach Anspruch 1, 2 oder 3, **dadurch gekennzeichnet, dass** bei dem Rotor (42) zwischen dem zweiten, expandierten Zustand in dem dritten Betriebszustand bezüglich der äußeren Form im Wesentlichen keine Unterschiede bestehen.

5. Rotor nach Anspruch 1, 2, 3 oder 4, **dadurch gekennzeichnet, dass** Fasern (10, 11, 13, 18, 19, 55, 56, 62, 63) vorgesehen sind, die sich in radialer Richtung über die Rotationsachse (14) hinaus erstrecken.

6. Rotor nach Anspruch 1, 2, 3, 4 oder 5, **dadurch gekennzeichnet, dass** im Betriebszustand wenigstens ein Teil der Fasern, insbesondere die Mehrheit der Fasern (10, 11, 13, 18, 19, 55, 56, 62, 63), abschnittsweise gerade verlaufen.

7. Rotor nach Anspruch 1, 2, 3, 4 oder 5, **dadurch gekennzeichnet, dass** im Betriebszustand wenigstens ein Teil der Fasern (10, 11, 13, 18, 19, 55, 56, 62, 63), insbesondere die Mehrheit der Fasern, entlang der Längsrichtung des jeweiligen Förderelementes (15) mit einer geringeren Krümmung verlaufen als die neutrale Faser (Nulllinie) und/oder neutrale Fläche des jeweiligen Förderelementes im Sinne der Festigkeitslehre.

8. Rotor nach Anspruch 1 oder einem der folgenden, **dadurch gekennzeichnet, dass** der Kunststoff des Rotors (42) eine Shore-Härte <100 D, insbesondere < 80 D, aufweist.

9. Rotor nach Anspruch 1 oder einem der folgenden, **dadurch gekennzeichnet, dass** ein erster Anteil von mehr als 30%, insbesondere mehr als 50%, der Fasern (10, 11, 13, 18, 19, 55, 56, 62, 63) im expandierten Zustand des Rotors (42) im Wesentlichen gestreckt von einem der Rotorachse (14) am nächsten liegenden Abschnitt (10a, 11a, 13a) zu einem der Rotationsachse (14) ferner liegenden zweiten Abschnitt (10b, 11b, 13b) verläuft.

10. Rotor nach Anspruch 1 oder einem der folgenden, **dadurch gekennzeichnet, dass** ein erster Anteil der Fasern (10, 11, 13, 18, 19, 55, 56, 62, 63), der mehr als 30% der Fasern, insbesondere mehr als 50%, der Fasern beträgt, eine Länge aufweist, die mindestens 30%, insbesondere mindestens 50%, der Maximalhöhe der Förderelemente (15), gemessen in Radialrichtung des Rotors (42) im expandierten Zustand, entspricht.

11. Rotor nach Anspruch 9 oder 10, **dadurch gekennzeichnet, dass** der erste Anteil der Fasern (10, 11, 13, 18, 19, 55, 56, 62, 63) im Wesentlichen senkrecht zur Rotationsachse (14) verläuft.

12. Rotor nach Anspruch 1 oder einem der folgenden, **dadurch gekennzeichnet, dass** der Durchmesser der Fasern (10, 11, 13, 18, 19, 55, 56, 62, 63), insbesondere des ersten Anteils der Fasern (10, 11, 13, 18, 19, 55, 56, 62, 63), kleiner als 40 Mikrometer ist.

13. Rotor nach Anspruch 1 oder einem der folgenden, **dadurch gekennzeichnet, dass** die Oberfläche der Fasern (10, 11, 13, 18, 19, 55, 56, 62, 63) mit einem Haftvermittler versehen ist.

14. Verfahren zur Herstellung eines Rotors (42) nach Anspruch 1 oder einem der folgenden mittels eines Gießverfahrens, insbesondere eines Spritzgießverfahrens, bei dem das Material der Förderelemente (15) in Radialrichtung in Bezug auf die Rotorachse (14) in die Volumina der Förderelemente derart eingebracht wird, dass der Gießwerkstoff in das Volumen jedes einzelnen Förderelementes jeweils in Radialrichtung (30, 31, 32, 33) einströmt,

15. Gießform für einen Rotor (42) gemäß einem der Ansprüche 1 bis 13, **dadurch gekennzeichnet, dass** bei den Volumina der Förderelemente (15) an deren radial verlaufenden Kanten (40, 41) Überströmkanäle vorgesehen sind, um einen störungsfreien Fluss des Gießwerkstoffs in Radialrichtung zu ermöglichen.
